# EUROPEAN PATENT SPECIFICATION

(11) **EP 1 346 727 B1**
(45) Date of publication and mention of the grant of the patent: **28.04.2010**
(21) Application number: 01998363.4
(22) Date of filing: 29.11.2001
(51) Int. Cl.: A61K 39/12, A61K 39/29, A61K 39/295, B01D 15/08

(54) **METHOD FOR OBTAINING DELIPIDATED HEPATITIS B ANTIGENIC AGGREGATES AND THEIR USE THEREOF**
VERFAHREN ZUR GEWINNUNG DELIPIDIERTER ANTIGENER AGGREGATE VON HEPATITIS B UND DEREN VERWENDUNG
METHODE D'OBTENTION D'AGGREGATS D'ANTIGENES DE L' HEPATITE B DELIPIDES ET LEUR UTILISATION

(30) Priority: 01.12.2000 CU 27900
(43) Date of publication of application: 24.09.2003
(73) Proprietor: CENTRO DE INGENIERIA GENETICA Y BIOTECNOLOGIA (CIGB), Ciudad de la Habana 10600 (CU)
(72) Inventor: AGUILAR RUBIDO, Julio, César, 32200 La Habana (CU); PENTON ARIAS, Eduardo, 12100 Ciudad de la Habana (CU); TLEUGULOVA, Dina, Cubanacan, Playa, Cludad de La Hbana (CU); SEWER MENSIES, Minerva, Bejucal, 32600 La Habana (CU); MUZIO GONZALEZ, Verena, Lucila, 10400 Ciudad de la Habana (CU); GUILL NENIETO, Gerardo, Enrique, 10400 Ciudad de la Habana (CU); SIMON BERNARD, Iloki, Assanga, 10900 Ciudad de la Habana (CU); CRUZ RICONDO, Luis, Javier, 11000 Ciudad de la Habana (CU); FALCON CAMA, Viviana, 10200 Ciudad de la Habana (CU); TAMBARA HERNANDEZ, Yanet, 10200 Ciudad de la Habana (CU)
(74) Representative: Prins, Adrianus Willem
(86) International application number: PCT/CU2001/000009
(87) International publication number: WO 2002/043756

(56) References cited:
- EP-A- 0 174 759
- EP-A- 0 231 039
- WO-A-01/98333
- BABAEVA E E ET AL: "ANTIGENIC DETERMINANTS OF NORMAL SERUM PROTEINS ASSOCIATED WITH HEPATITIS B VIRUS HEPATITIS B SURFACE ANTIGEN" VOPROSY VIRUSOLOGII, num. 4, 1981, páginas 442-446, XP002209718 ISSN: 0507-4088

## Description

The present invention is related to the medical branch, particularly with the use of new vaccine formulations.

The technical objective of the proposed invention is the development of a method for the preparation of aggregated antigenic structures and their formulations, able to potentiate the immunogenicity of the present antigens administered by systemic or mucosal routes.

EP 0 231 039 describes a process for preparing immunogenic complexes useful as a vaccine.

EP 0 174 759 discloses particles of protein aggregates useful as a vaccine.

The method described in the present invention generates antigenic aggregated structures of particulated antigens, the addition of other antigens, components with aggregating, delipidating or oxidizing characteristics, the post-selection of aggregated particles between 30 - 500 nm size, and the formulation of those aggregates conveniently adjuvanted, favor the immunogenicity of the resulting antigenic composition.

Since the HBsAg is an efficient immunogen, it was the first vaccine candidate of a wide use in humans and the first licensed anti hepatitis B recombinant vaccine for universal use. HbsAg proteins are self-assembling in 22 nm particles (Heerman KH, Gerlich WH, 1991 Surface protein of Hepatitis B virus. A. McLachlan, ed. CRC Press, Boca Raton, Ann Harbor, Boston, London, p.109). The molecular, cellular and genetic basis of the immune response to HBsAg has been extensively studied in a murine model (Milich, DR. 1987. Genetic and molecular basis for T- and B- cell recognition of hepatitis B viral antigens. Immunol Rev. 99: 71). Formerly it had been studied how the variations of the physical chemical properties of the surface antigen affect its immunogenicity for MHC class I restricted T cells. It was evidenced that an efficient MHC class I restricted CTL response was generated with only one low dose injection of the 22 nm particulated native antigen or with detergent denatured monomers without adjuvant (Schirmbeck, R. et al. 1994. Eur J Immunol. 24: 1088). It has also been investigated the immunogenicity of a preparation of aggregates of the HBV surface antigen obtained by heat denaturation to produce particles of approximately 1 µm diameter.

The type of *in vivo* antigenic processing has a fundamental influence on the efficiency of the primary T cell response as well as on the subclass spectrum of the involved T cell. In the presentation of peptides to the MHC apparently operates alternative processes for MHC class II CD4+ restricted T cells and for MHC class I CD8+ restricted T cells (Germain, RN. et al. 1993. Annu Rev Immunol. 11: 403). It has been described a new endosomal pathway for the exogenous HBsAg particle processing to present MHC class I restricted epitopes. This heat denatured 1 µm diameter exogenous HBsAg particle processing occurs in macrophages but not in other cell types and is accompanied with regurgitation of antigenic peptides from the processing macrophage. This exogenous antigen MHC class I restricted peptide generating process has been tentatively designed as the phagocytic pathway. The native 22 nm HBsAg particles are processed mainly by the endocytic and the 1 µm aggregates by the phagocytic pathways. In addition to the different *in vitro* processing of these two HBsAg exogenous preparations, their *in vivo* immunogenicity for class I CTL was markedly different when they were sent without adjuvants. The native HBsAg particle was highly immunogenic while the denaturalized surface antigen aggregates were of low immunogenicity (Schirmbeck, R. et al. 1995. J Immunol 155: 4676-4684).

Other studies performed using fluorescence polarization suggest that the HBsAg particle is organized as a lipid bilayer which interacts with protein aggregates (Sonveaux N. 1995 Res Virol 146 (1):43-51).

The HBsAg treatment with chloroform-methanol (2:1, v/v) 50% 1,1',3,3'-tetrametilurea did not affect the morphologic integrity of the particles (they maintained their mean diameter), although a major portion of its lipids was released. The antigenicity and the HBsAg polypeptide composition was not altered by the delipidation (Neurath AR et al 1978 Intervirology 10(5): 265-75).

Taking into account that particle aggregations are not produced in the stressing conditions of the production process, such as the presence of chaotropic agents, moderate temperatures and highly concentrated solutions, the source of recombinant hepatitis B surface antigen particles aggregation has been studied using a combination of immunoaffinity and molecular size exclusion chromatography techniques. The investigation of factors conducing to an increase in the base of the peak corresponding to HBsAg in molecular exclusion chromatography demonstrated the existence of particle aggregates, in addition to the size variability of the HBsAg particle (Tleugabulova D. 1998 J Chromatogr B Biomed Sci Appl 707(1-2):267-73, Tleugabulova D. 1997 Chromatographia 45: 317-320). As a result the aggregated antigen was obtained in the fraction corresponding to the large particle aggregates was obtained and not in the fraction were the native HBsAg protein was found, another result of this article corroborates that the aggregates are formed by 22 nm particles migrating as monomers and dimers in SDS-PAGE as well as the correctly folded surface antigen (Tleugabulova D, et al. 1998 J Chromatogr B Biomed Sci Appl 25;716(1-2): 209-19).

### Disclosure of the invention

One of the objectives of the present invention is a method to obtain aggregated antigenic structures of a higher immunogenicity than the antigens originating them. Said method includes the following steps:
A) Selection of the antigens of interest;
B) Addition of one or several antigens of the mixture in a medium favoring the aggregation process, said medium may consist in chemical agents, oxidizing agents or other components with aggregating capacity.
C) Incubation of the mixture.
D) Selection of the particle aggregate with a size between 30 and 500 nm by a process allowing the retention of these molecular sizes, such as molecular exclusion chromatography, diafiltration and dialysis.
E) Preparation of the formulations by mixing the selected antigenic structures in step (C) through the addition of the adjuvant of election and also the potential addition of other antigens, stabilizers and preservatives.

Several aggregates that only contain the surface antigen of the HBV may be obtained by the present invention method, including also combinations of the surface antigen and other lipoprotein, lipopeptide or lipidic homologous or heterologous antigens from any viral, bacterial, unicellular or multicellular pathogen.

According to the invention method the antigens that are part of the structures obtained in the step (B) may be added to the surface antigen of the hepatitis B virus by hydrophobic, electrostatic or covalent interactions, generating aggregates of different sizes.

The method to obtain antigenic structures allows the aggregation of the hepatitis B, C or HIV nucleocapsid antigens to the hepatitis B virus surface antigen, also antigens from virus and bacteria such as inactivated virus or outer membrane proteins of bacterial pathogens like *Neisseria meningitidis,* may be aggregated.

To obtain the aggregated antigenic structures β-cyclodextrins may be used as chemical agent favoring delipidation, membrane association and aggregation of the present particles, other chemical agents are ammonia salts at concentrations between 10 and 50 mM, potentiated by metal salts of copper and iron and others permitting aggregation.

Other components of the method presenting spontaneous aggregating activity that may be used together or alone for this purpose are the homologous or heterologous antigens that evidenced aggregating activity on HBsAg, among them the viral nucleocapsid antigens and also bacterial outer membrane derivatives or viral envelopes of lipoprotein or hydrophobic nature. It was also found that adjuvants of the same nature favored HBsAg aggregation and that of HBsAg with themselves.

In general the present invention method allows HBsAg aggregation or the HBsAg aggregation with other antigens or adjuvants through the development of hydrophobic interactions, electrostatic or covalent linkages, with incubation periods ranking from 10 minutes to one week, depending on the selected constituents.

The aggregates separation is achieved by molecular size exclusion methods, among them molecular exclusion chromatography, dialysis, diafiltration or other method permitting the retention of molecular sizes between 30 and 500 nm.

We have also shown that although it is possible to generate aggregates of around 1 micrometer of size by non denaturing methods (maximum temperature 28°C), similar to those obtained by Schirmbeck (Schirmbeck, R. et al. 1995. J Immunol 155: 4676-4684), these continue to be less immunogenic when evaluating the humoral response and DTH. Only those particles aggregates of a size between 30 and 500 nm, adjuvanted with alum, have demonstrated the generation of IgG levels significantly higher than the native antigen control, showing additionally a significant increase of the DTH and IgG2a responses. All this evidences the importance of the later selection of the antigen by molecular exclusion chromatography. The cause of this performance may be given by the different presentation and or processing of the antigen or antigens of interest.

Moreover the present invention method foresees the adsorption of the resulting structures to such adjuvants as alum or calcium salts, oily or other commercially used adjuvant. It may be also added to the final formulation other antigens and stabilizing and preserving substances.

Another object of the present invention is the aggregated antigenic structure obtained according to the previously described method, which favors an increase in the immunogenicity of the resulting formulation and a differential recognition by the immune system of the involved epitopes. Said aggregated antigenic structures are characterized by the presence of the hepatitis B virus surface antigen, alone or in combination with other antigens forming the aggregate. These other antigens are lipoproteins or hydrophobic, among them HBcAg, possessing additionally the intrinsic property of favoring the aggregation state between them by hydrophobic linkages. Other hydrophobic viral capsid and lipoprotein antigens have shown this capacity, among them the nucleocapsid antigen of the hepatitis C virus, the human papilloma virus and HIV 1 and 2, besides the outer membrane of *N. meningitidis* in proteolyposome vesicles and some viral envelope antigens.

Among the antigenic structures object of the present invention the associations of HBsAg with hydrophobic adjuvants are included which may be part of the aggregate by the same previously described method. In general the antigenic structures object of the present invention may be obtained by aggregation of at least one, two or more hydrophobic particles according to the described method and at least one of particulate character, and should be visible by electron microscopy as described in the examples. The aggregation of these structures favors the immune modulation, differential recognition and immunogenicity enhancement in a general fashion. Taking into account these characteristics of the antigenic structures object of the present invention, it is possible the use them for the rational design of preventive and therapeutic human and veterinary vaccines, through the systemic or mucosal routes and their use in diagnostic systems.

Among the advantages of the new preparations resulting from the use of the method for obtaining this type of antigens the following are found: increase of immunogenicity, co-trapping capacity for new adjuvants, immunomodulators and antigens during the aggregation.

The preparations resulting from the present invention method, depending on the inoculation route and species to be immunized may be used in volumes of 0.01 up to 10 mL and the antigen doses may vary between 0.001 and 1 mg in the final vaccine formulation.

### EXAMPLES

### Example 1. Preparation of vaccine formulations of aggregated of surface antigen of the hepatitis B virus obtained by the cyclodextrins use.

The particles were obtained from the 22 nm native antigen by the controlled treatment of the antigen with chemical compounds with lipid subtracting activity from the particle, in this case cyclodextrins were used in concentrations higher than 1 mg/mL. Depending on its concentration the incubation time varied from 24 hours to 7 days. The incubation temperature used in this assay was of 28 degrees centigrade although it has been observed that at higher and lower temperatures it is also possible to obtain aggregates. The temperature is a factor favoring the partial delipidation process though oxidation and lipids subtraction. Later the different aggregates were analyzed by gel filtration and electron microscopy, finding sizes which varied from tenths nanometers up to particles that precipitated due to their huge size. After centrifugation to eliminate precipitated residues, the antigen was selected depending on its size for immunochemical analyses, which demonstrated a decreased level of lipids regarding the proteins level. It has been shown by HPLC that these aggregates have a high stability during the storage time. The controlled treatment of the antigen with β-cyclodextrins, between 5 - 100 mg/mL during 1 - 240 hours, at temperatures ranging from 20 to 37 °C, allow to obtain a size range that made possible the later election of the elution time for immunochemical analysis.

Afterwards the aggregated antigen was adsorbed to alum at a final concentration between 0.002 and 0.1 mg/mL and was used for immunogenicity assays.

One incubation variant with cyclodextrins at different times and temperatures involved the addition of immunomodulating compounds such as lypo-polysaccharides and saponins, that are part of the final aggregate adsorbed to alum to produce the final vaccine formulation.

### Example 2. Preparation of vaccine formulations of surface antigen of hepatitis B virus aggregates using oxidizing agents.

With the addition of oxidizing chemical substances to the normal antigen it was possible the delipidation in controlled time, temperature and concentration conditions in the same way that with cyclodextrins. Salts as ammonium peroxi-disulfate between 9 and 44 mM, permitted the generation of the delipidated antigen starting from the 22 nm antigen particles, to produce size increase by particle fusion. The optimal sizes were selected by gel filtration. La adjuvant adsorption was achieved on alum.

In the same way that in the example 1, it was possible to include in the aggregate different quantities of other adjuvants and immunomodulators during the incubation.

### Example 3. Preparation of vaccine formulations of over-particulate chemical structures, obtained by modification of the incubation conditions of the yeast.

The particles were obtained naturally from the *Picchia pastoris* yeast strain, the antigen was selected during the purification process by its physical chemical characteristics. The antigen production process is submitted to long lasting culture time, higher than 100 hours and oxidative stressing conditions. This process makes possible that a part of the antigen remains in its 22 nm particle size native state but an important moiety gets aggregated and delipidated by the increase of the intracellular oxidative conditions, as demonstrated by the lipid and protein analyses done to samples of the different peaks from gel filtration. Finally the fraction is separated by HPLC gel filtration in TSK G5000 columns. This material reaches up to 10% of total the antigen which is actually discarded. Alum adjuvant adsorption is achieved in similar conditions as for the normal antigen.

The analysis of both antigens demonstrated that HBsAg gets aggregated in a process that involves a significant loss of lipids of all types which is shown for phospholipids of both antigens in the following table:

**Composition of HBsAg phospholipids (PL) separated by silica-gel.**

| ng PL/µg of protein | HBsAg (50-500 nm) | HBsAg 22 nm |
|---|---|---|
| Total phospholipids | 285.6 ± 81.9 | 1225.3 ± 256.8 (**) |
| Phosphatidilcholine | 109.4 ± 47.6 | 779.3 ± 168.3 (**) |
| Lysophosphatidilcholine | 20.0 ± 12.7 | 73.5 ± 26.1 (**) |
| Phosphatidilethanolamine | 52.8 ± 13.3 | 183.7 ± 54.3 (**) |
| Phosphatidilserine | 28.8 ± 11.1 | 78.4 ± 26.0 (**) |
| Phosphatidilinositol | 25.3 ± 10.1 | 74.7 ± 18.8 (**) |
| Phospholipids bound to HBsAg | 48.8 ± 10.7 | 41.1 ± 7.7 (NS) |

With this example it is evidenced that a new over-particulated antigen may be obtained after a natural oxidizing and delipidating process. The stability of these aggregates would be based on the aggregation processes that may occur during the elimination of lipids from the particles that could expose hydrophobic regions and by protein polymerizations between particles when sulphydril groups are exposed.

### Example 4. Evaluation of the aggregated HBsAg immunogenicity.

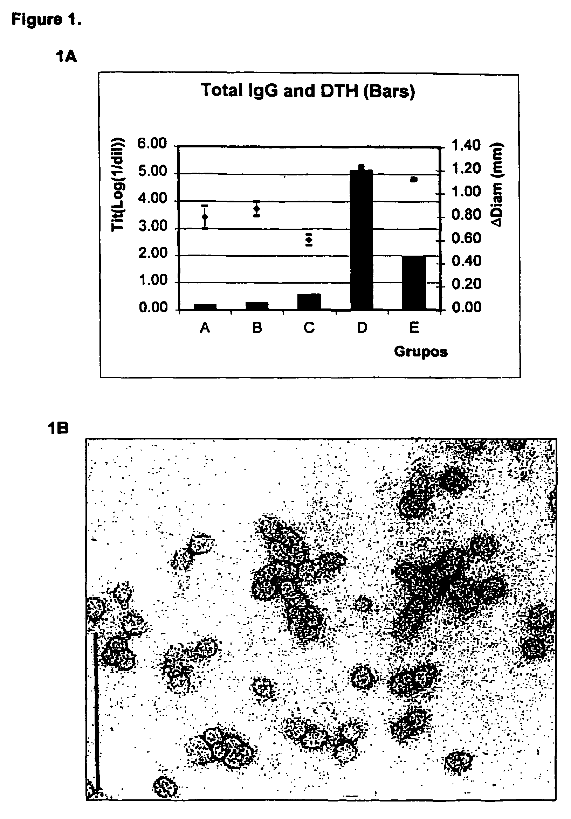

With the objective of evaluating the immunogenicity of the aggregated HBsAg resulting from the example 3, adsorbed in alum or in PBS for mucosal route, an immunization schedule was carried out with inoculations the days 0, 14 and 28 and retro-orbital blood extractions the day 42 and female Balb/c mice of 10 to 15 weeks old were immunaized by intranasal and intramuscular routes. The doses per mouse are presented in the table at the end of this example, and the results are shown in figure 1A, and the HbsAg aggregate are shown in figure 1B.

The statistical analysis of results was performed by the Student test and p<0.05 was considered a significant difference.

From this experiment it was demonstrated that it is possible to generate a higher anti HBsAg IgG response when immunizing by mucosal or systemic routes, regarding the normal antigen, in equal conditions. In the same figure it is represented the comparison of this effect for the DTH response (bars), which also resulted significantly higher for the aggregated variant.

The immunization groups are shown in the following table:

| | | |
|---|---|---|
| A- | 5 µg HBsAg delipidated (50-500 nm)/ PBS 1X | IN |
| B- | 10 µg HBsAg delipidated (50-500 nm)/ PBS 1X | IN |
| C- | 5 µg HBsAg normal (22 nm)/ PBS 1X | IN |
| D- | 5 µg HBsAg delipidated (50-500 nm)/ Alum 0.5 mg/mL | IM |
| E- | 5 µg HBsAg normal (22 nm)/ Alum 0.5 mg/mL | IM |

### Example 5. Kinetics of the anti-HBsAg IgG response.

With the objective of studying the kinetics of the anti-HBsAg IgG response 10 groups of female 10 - 15 weeks old Balb/c mice were immunized. The schedule used was: inoculation the weeks 0, 2 and 18 and extractions pre immune at 4, 6, 8, 10, 12, 14, 16 y 20 weeks. The groups tested are described in the following table:

| | | |
|---|---|---|
| 1- | 5 µg HBsAg normal (22 nm)/ Alum | IM |
| 2- | 5 µg HBsAg normal (22 nm)/ PBS 1X | IM |
| 3- | 5 µg HBsAg delipidated (example 3)/ Alum 0.5 mg/mL | IM |
| 4- | 5 µg HBsAg delipidated (example 3) / PBS 1X | IM |
| 5- | 5 µg HBsAg delipidated (example 1) / Alum 0.5 mg/mL | IM |
| 6- | 5 µg HBsAg delipidated (example 1) / PBS 1X | IM |
| 7- | 5 µg HBsAg delipidated (example 1) /Alum 0.5 mg/mL | IM |
| 8- | 5 µg HBsAg delipidated (example 1)/ PBS 1X | IM |
| 9- | 5 µg HBsAg delipidated (example 1)/ Alum 0.5 mg/mL | IM |
| 10- | 5 µg HBsAg delipidated (example 1)/ PBS 1X | IM |

The antigens of the groups 5 and 6, 7 and 8, and 9 and 10 were obtained by incubating at different times and concentrations of cyclodextrins obtaining different degrees of aggregation.

For the DTH experiment, the measurements were performed the days: 1(1, 3), 2(1', 3'), 3(1", 3") and 5(1"', 3"')

The statistical analysis of results was performed by the Student test: p<0.05 se was considered a significant difference.

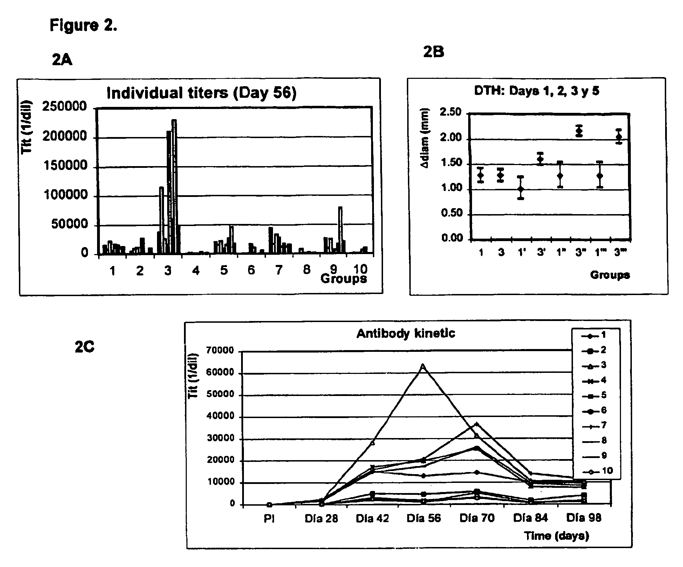

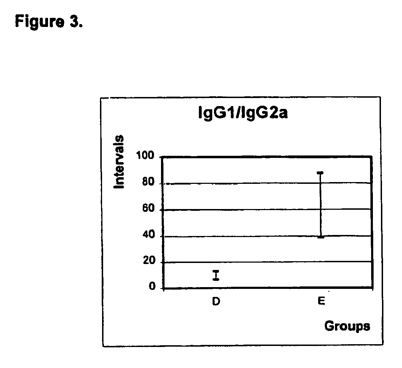

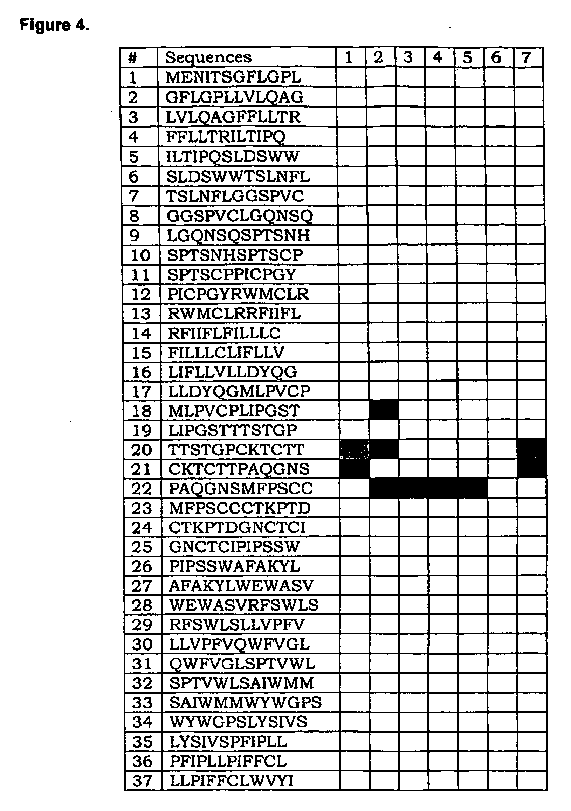

In this experiment it was corroborated that the delipidated HBsAg, with different degrees of aggregation, have immunological characteristics that distinguished each one. The larger immunogenicity and DTH response did not corresponded to the larger antigen size but to intermediate antigen sizes although according to the performance of the antibody appearance kinetics at the end of the experiment the larger immunogenicity corresponded to the variant with a higher degree of aggregation. However it was observed a significant increase of the DTH response in the groups immunized with alum adjuvant as compared to those immunized with the antigen in PBS, the group of aggregated antigen with an intermediate size generated the larger increases with arrived to be significant the day 56. In figure 2a the individual titers the day 56 are represented for all immunized groups. In this figure are also represented the results of the DTH experiment performed during 5 days of the week 22, using 20 µg of HBsAg in the right leg and PBS in the left leg in an inoculation volume of 20 µL. All resting groups also had a significantly lower response regarding the difference in diameter between the right and left legs as compared to group 3, which was inoculated with alum adjuvanted HBsAg with an intermediate aggregation level, obtained as described in example 3. It is worth to take into account that in the later examples it is demonstrated that the recognition in the case of over-particulated structures is wider regarding the epitopes recognized in the HBsAg, which indicates a better performance for this type of structure. Although from group 5 to group 10, the anti native HBsAg reactivity is similar to that obtained immunizing with native HBsAg during a major part of the time, a strong response is also obtained against other epitopes present in the aggregated antigen, see example 8, figure 4.

### Example 6. Evaluation of the IgG1/IgG2a ratio.

With the objective of studying if a variation in the IgG1/flgG2a ratio existed due to the its linkage between the TH1/TH2 response, the sera of the D and E groups of the immunization schedule of example 4, were tested for anti IgG 1 and IgG2a antibody levels. This analysis was performed for the sera of the day 42 extraction. The IgG2a antibody levels increased significantly in the group immunized with the over-particulated antigen (50-500 nm) up to attaining an IgG1/IgG2a ratio closer to 1 as compared to the normal HBsAg also adjuvanted in alum (Figure 4).

The IgG1/2a ratio for the normal HBsAg group immunized with alum was 6.2 times higher to that found for the delipidated HBsAg group of intermediate size. From this experiment it could be concluded that the different presentation of the surface antigen not only generates a quantitative but also a qualitative change regarding the IgG type which is potentiated and the correlation of these variations in the IgG subclass pattern with an enhancement of the cellular response, corroborating the DTH assessment findings.

### Example 7. Study of the anti- native HBsAg reactivity of the sera of mice immunized with different antigenic variants.

After the immunization of mice with the delipidated antigens obtained according to examples 1, 2 and 3, the reactivity of their sera was compared to that of the sera from the mice immunized with the normal antigen. As a result of this experiment it was observed that the immune response generated in the sera from the mice immunized with the different variants had a different reactivity against the normal HBsAg (22 nm), the major reactivity was exhibited by the sera from mice immunized with the normal antigen, while for the different degrees of delipidation the reactivity against HBsAg was also different. Even existing high titers against their own immunogens the sera from mice immunized with immunized with highly oxidized variants did not recognized HBsAg, evidencing a different recognition of the generated antibodies with the different immunogens and demonstrating the different antigenic nature of the newly generated structures. Therefore the results of example 5 must be analyzed taking into account that although for the last three aggregated antigen variants similar anti native HBsAg responses are obtained, a response is also present against other HBsAg protein epitopes not recognized by immunizing with native HBsAg.

### Example 8. Recognition of lineal epitopes by the sera from mice immunized with different antigen variants.

With the objective of comparing the recognition of the antibodies generated by an over-particulated HBsAg obtained by the oxidation variant described in example 2, a mapping was performed on cellulose membrane containing linear HBsAg sequences (S region), 37 peptides of 12 aminoacids were synthesized each one overlapped in 6 until completing the whole protein sequence.

The epitopic mapping on cellulose membrane was performed according to Ronald Frank (Frank, R. 1992 Tetrahedron 48: 9217-9232). The serum samples were assayed at a 1/100 dilution.

Results of facing the sera from the mice inoculated with the normal antigen and the different aggregated variants of the same antigen, evidenced that there is no a similar linear recognition pattern between both antigens, which leads to the conclusion that a different presentation is produced for the B epitopes present on the surface of HBsAg and its aggregated variants (figure 4).

### Example 9. Formation of aggregates between HBsAg and nucleocapsid antigens. Immunological assessment.

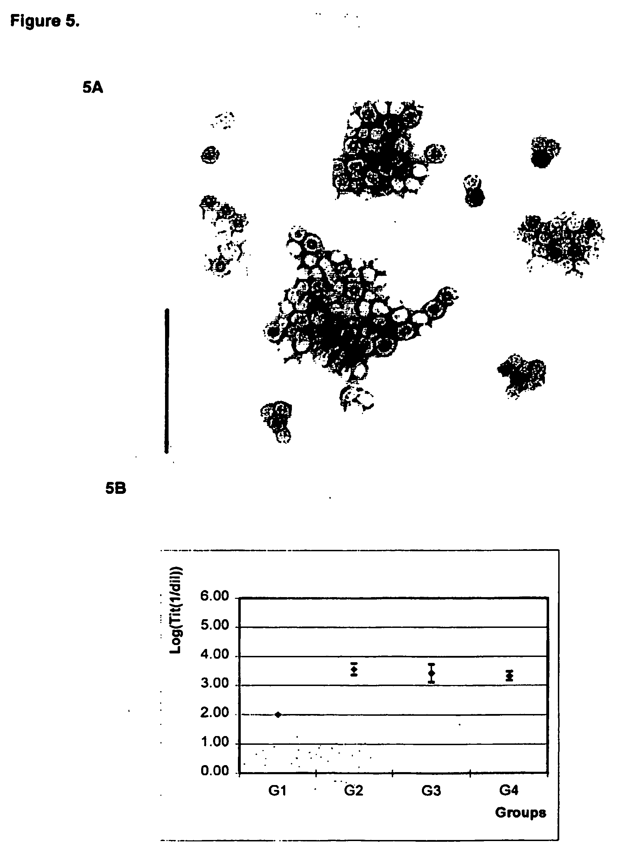

Equal volumes of two preparations containing 0.1 mg/mL HBsAg and HBcAg were incubated at 4°C overnight and afterwards aggregates were obtained by HPLC TSK G6000 molecular exclusion chromatography. A sample of these aggregates was processed for electron microscopy visualization techniques, the other sample was used for its immunological evaluation with an immunization schedule in Balb/C mice by intranasal inoculation, with both antigens separately and with the aggregate verified by electron microscopy (Fig. 5A).

Results evidenced that the mixture of both aggregated antigens by intranasal route allowed the potentiation of the response against the HBsAg (Fig 5B). The groups of the figure are represented in the following table:

| | |
|---|---|
| 1-10 µg HBsAg/ PBS 1X | IN |
| 2-10 µg HBsAg/ acemannan 3 mg/mL . | IN |
| 3-10 µg HBsAg/ 10µg HBcAg / PBS 1X | IN |
| 4-10 µg HBsAg/ Alum 0.5 mg/mL | SC |

Similar results were obtained when other viral capsid antigens such as hepatitis C and HIV were used to prepare the mixture.

### BRIEF DESCRIPTION OF THE DRAWINGS

**Figure 1****.**
   (A) Three dose schedule (0, 2 y 4 weeks). The extractions were performed the week 6. The three first groups were inoculated with 50 µL by intranasal route. The two remaining groups were inoculated by intramuscular route in alum with 100 µL.
   (B) Aggregated HBsAg. (meter: 200nm)
**Figure 2****.**
   Three dose schedule (0, 2 y 18 weeks). All groups were inoculated by intramuscular route with a volume of 100 µL. The antibody values correspond to the week 8 (fig. 2a). During the week 22 se the DTH test was performed. Figure 2b represents the DTH value for groups 1 and 3 during 5 days. Figure 2c represents the kinetics of increase of titers during the schedule.
**Figure 3****.**
   The same 3 doses schedule (0, 2 y 4 weeks) of figure 1. Analysis of groups D and E sera from the week 6 extraction. Both groups were inoculated by intramuscular route in alum with 100 µL, group D with delipidated HBsAg (50-500 nm) and group E with normal HBsAg with the same doses. In the graph it is represented the geometric mean value and interval of the IgG1/lgG2a ratio for each mouse of both groups.
**Figure 4****.**
   Mapping of overlapping peptides of the S region of HBsAg on cellulose membrane containing linear sequences. 1/100 dilutions of sera pools were analyzed: 1, HBsAg (normal): 2, HBsAg (aggregated); (3-5: monoclonal antibodies (MAbs) obtained immunizing with aggregated antigens; 3, clone 6; 4, clone 7; 5, clone 8); 6, non related serum; 7, MAb (Hep 1). Four color intensities represent the response against the peptides. Blank: negative response, clear gray: slightly positive response, dark gray: positive response, black: very positive response.
**Figure 5****.**
   (A) Electron microscopy of HBsAg aggregates and HBcAg, the higher electron-dense particles in the center mayor corresponds to HBcAg, the other are HBsAg.
   (B) 2 dose schedule (0, 14 days). Analysis of sera the day 26.

## Claims

1. A method for preparing highly immunogenic aggregated antigenic structures, which comprises the following steps:
A) selection of the antigens of interest;
B) addition of one or several antigens of the mixture in a medium which favors aggregation;
C) incubation of the mixture;
D) delipidation of the mixture;
E) selection of particle aggregates having a size of from 30 to 500 nm using a process which permits the retention of these molecular sizes; and optionally,
F) preparation of formulations from the mixture of the antigenic structures selected in step (E) by addition of an adjuvant of choice and optional addition of other antigens, stabilizing agents and preserving agents;
wherein the antigens of interest comprise Hepatitis B surface Antigen (HBsAg) optionally in combination with other antigens.

2. A method according to claim 1, wherein the antigens of interest comprise HBsAg only.

3. A method according to claim 1, wherein the antigens of interest comprise HBsAg in combination with other antigens.

4. A method according to claim 3, wherein the other antigens are selected from the group consisting of Hepatitis B Virus nucleocapsid antigen (HBcAg), Hepatitis C Virus nucleocapsid antigen, Human Immunodeficiency Virus type 1 or 2 nucleocapsid antigen, and Neisseria meningitidis outer membrane protein.

5. A method according to any one of the preceding claims, wherein the medium used in step (B) comprises chemical agents, oxidizing agents or other components having aggregating capacity.

6. A method according to claim 5, wherein the medium used in step (B) comprises β-cyclodextrins.

7. A method according to claim 5 or 6, wherein the medium used in step (B) comprises ammonium salts in a concentration of 10-50 mM, potentiated by metal salts of copper and iron and others permitting aggregation.

8. A method according to any one of claims 5-7, wherein the medium used in step (B) comprises adjuvant of total or partial hydrophobic nature which favors HBsAg aggregation.

9. A method according to any one of the preceding claims, wherein the incubation step (C) takes from 10 minutes to one week.

10. A method according to any one of the preceding claims, wherein in step (E) particle aggregates of a size from 50 to 500 nm are selected.

11. A method according to any of the preceding claims, wherein the adjuvant of choice used in step (F) is an aluminium or calcium salt adjuvant or an oily adjuvant.

12. A highly immunogenic aggregate of antigenic particles prepared according to the method of any one of the preceding claims, comprising Hepatitis B surface Antigen (HBsAg) optionally in combination with other antigens, wherein the particle aggregate has a size of from 30 to 500 nm.

13. An aggregate according to claim 12, wherein the antigens comprise HBsAg only.

14. An aggregate according to claim 12, wherein the antigens comprise HBsAg in combination with other antigens.

15. An aggregate according to claim 14, wherein the other antigens are selected from the group consisting of Hepatitis B Virus nucleocapsid antigen (HBcAg), Hepatitis C Virus nucleocapsid antigen, Human Immunodeficiency Virus type 1 or 2 nucleocapsid antigen, and Neisseria meningitidis outer membrane protein.

16. An aggregate according to any one of the claims 12 to 15, wherein the aggregate comprises adjuvant of total or partial hydrophobic nature.

17. A vaccine formulation comprising a highly immunogenic aggregate as defined in any one of the claims 12 to 16, together with adjuvant, stabilizing agent or preserving agent.

18. A vaccine formulation according to claim 17, for systemic or mucosal application.

## Patentansprüche

1. Verfahren zum Herstellen von hochimmunogenen aggregierten antigenen Strukturen, welches die folgenden Schritte umfasst:
A) Auswahl der Antigene von Interesse;
B) Zugabe von einem oder mehreren Antigenen der Mischung in ein Medium, weiches eine Aggregation begünstigt;
C) Inkubation der Mischung;
D) Delipidierung der Mischung;
E) Auswahl von Teilchenaggregaten mit einer Größe von 30 bis 500 nm unter Verwendung eines Verfahrens, welches die Retention dieser Molekülgrößen gestattet; und gegebenenfalls,
F) Herstellung von Formulierungen aus der Mischung der in Schritt (E) ausgewählten antigenen Strukturen durch Zugabe eines Adjuvans der Wahl und gegebenenfalls Zugabe von anderen Antigenen, Stabilisierungsmitteln und Konservierungsmitteln ;
wobei die Antigene von Interesse Hepatftis-B-Oberflächenantigen (HBsAg), gegebenenfalls in Kombination mit anderen Antigenen, umfassen.

2. Verfahren nach Anspruch 1, wobei die Antigene von Interesse nur HBsAg umfassen.

3. Verfahren nach Anspruch 1, wobei die Antigene von Interesse HBsAg in Kombination mit anderen Antigenen umfassen.

4. Verfahren nach Anspruch 3, wobei die anderen Antigene ausgewählt sind aus der Gruppe bestehend aus Hepatitis-B-Virus-Nucleocapsid-Antigen (HBcAg), Hepatitis-C-Virus-Nucleocapsid-Antigen, Humanes-Immundefizienz-Virus-Typ 1 oder 2-Nucleocapsid-Antigen und Neisseria meningitidis-Außenmembranprotein.

5. Verfahren nach einem der vorangehenden Ansprüche, wobei das in Schritt (B) verwendete Medium chemische Mittel, Oxidationsmittel oder andere Komponenten mit einem Aggregationsvermögen umfasst.

6. Verfahren nach Anspruch 5, wobei das in Schritt (B) verwendete Medium β-Cyclodextrine umfasst.

7. Verfahren nach Anspruch 5 oder 6, wobei das in Schritt (B) verwendete Medium Ammoniumsalze in einer Konzentration von 10-50 mM umfasst, verstärkt durch Metallsalze von Kupfer und Eisen und andere, die eine Aggregation gestatten.

8. Verfahren nach einem der Ansprüche 5-7, wobei das in Schritt (B) verwendete Medium ein Adjuvans mit vollständig oder teilweise hydrophober Beschaffenheit umfasst, welches die HBsAg-Aggregation begünstigt.

9. Verfahren nach einem der vorangehenden Ansprüche, wobei der Inkubationsschritt (C) 10 Minuten bis eine Woche dauert.

10. Verfahren nach einem der vorangehenden Ansprüche, wobei in Schritt (E) Teilchenaggregate mit einer Größe von 50 bis 500 nm ausgewählt werden.

11. Verfahren nach einem der vorangehenden Ansprüche, wobei das in Schritt (F) verwendete Adjuvans der Wahl ein Aluminium- oder Calciumsalz-Adjuvans oder ein öliges Adjuvans ist.

12. Hochimmunogenes Aggregat von antigenen Teilchen, hergestellt gemäß dem Verfahren nach einem der vorangehenden Ansprüche, umfassend Hepatitis-B-Oberflächenantigen (HBsAg), gegebenenfalls in Kombination mit anderen Antigenen, wobei das Teilchenaggregat eine Größe von 30 bis 500 nm aufweist.

13. Aggregat nach Anspruch 12, wobei die Antigene nur HBsAg umfassen.

14. Aggregat nach Anspruch 12, wobei die Antigene HBsAg in Kombination mit anderen Antigenen umfassen.

15. Aggregat nach Anspruch 14, wobei die anderen Antigene ausgewählt sind aus der Gruppe bestehend aus Hepatitis-B-Virus-Nucleocapsid-Antigen (HBcAg), Hepatitis-C-Virus-Nucleocapsid-Antigen, Humanes-Immundefizienz-Virus-Typ 1 oder 2-Nucleocapsid-Antigen und Neisseria meningitidis-Außenmembranprotein

16. Aggregat nach einem der Ansprüche 12 bis 15, wobei das Aggregat ein Adjuvans mit vollständig oder teilweise hydrophober Beschaffenheit umfasst.

17. Impfstoffformulierung umfassend ein hochimmunogenes Aggregat wie in einem der Ansprüche 12 bis 16 definiert, zusammen mit einem Adjuvans, Stabilisierungsmittel oder Konservierungsmittel.

18. Impfstoffformulierung nach Anspruch 17 für eine systemische oder mukosale Applikation.

## Revendications

1. Procédé pour préparer des structures antigéniques agrégées hautement immunogènes, qui comprend les étapes suivantes :
A) sélection des antigènes d'intérêt ;
B) addition d'un ou de plusieurs antigènes du mélange dans un milieu qui favorise une agrégation ;
C) incubation du mélange ;
D) délipidation du mélange ;
E) sélection d'agrégats particulaires ayant une taille comprise entre 30 et 500 nm en utilisant un processus qui permet la rétention de ces tailles moléculaires ; et facultativement,
F) préparation de formulations à partir du mélange des structures antigéniques sélectionnées à l'étape E), par l'addition d'un adjuvant de choix et l'addition facultative d'autres antigènes, d'agents stabilisants et d'agents conservateurs ;
où les antigènes d'intérêt comprennent l'antigène de surface de l'hépatite B (HBsAg), facultativement en combinaison avec d'autres antigènes.

2. Procédé selon la revendication 1, dans lequel les antigènes d'intérêt comprennent HBsAg uniquement.

3. Procédé selon la revendication 1, dans lequel les antigènes d'intérêt comprennent le HBsAg en combinaison avec d'autres antigènes.

4. Procédé selon la revendication 3, dans lequel les autres antigènes sont sélectionnés dans le groupe consistant en l'antigène de la nucléocapside du virus de l'hépatite B (HBcAg), l'antigène de la nucléocapside du virus de l'hépatite C, l'antigène de la nucléocapside du virus de l'immunodéficience humaine de type 1 ou 2, et la protéine de la membrane externe de *Neisseria meningitidis.*

5. Procédé selon l'une quelconque des revendications précédentes, dans lequel le milieu utilisé à l'étape (B) comprend des agents chimiques, des agents oxydants ou d'autres composants ayant une activité d'agrégation.

6. Procédé selon la revendication 5, dans lequel le milieu utilisé à l'étape (B) comprend des β-cyclodextrines.

7. Procédé selon la revendication 5 ou 6, dans lequel le milieu utilisé à l'étape (B) comprend des sels d'ammonium en une concentration de 10-50 mM, potentialisés par des sels métalliques de cuivre et de fer et autres permettant une agrégation.

8. Procédé selon l'une quelconque des revendications 5-7, dans lequel le milieu utilisé à l'étape (B) comprend un adjuvant ayant une nature hydrophobe totale ou partielle qui favorise l'agrégation du HBsAg.

9. Procédé selon l'une quelconque des revendications précédentes, dans lequel l'étape d'incubation (C) dure 10 minutes à une semaine.

10. Procédé selon l'une quelconque des revendications précédentes, dans lequel à l'étape (E) des agrégats particulaires ayant une taille comprise entre 50 et 500 nm sont sélectionnés.

11. Procédé selon l'une quelconque des revendications précédentes, dans lequel l'adjuvant de choix utilisé à l'étape (F) est un adjuvant d'aluminium ou de sel de calcium ou un adjuvant huileux.

12. Agrégat de particules antigéniques hautement immunogène préparé selon le procédé de l'une quelconque des revendications précédentes, comprenant l'antigène de surface de l'hépatite B (HBsAg) facultativement en combinaison avec d'autres antigènes, où l'agrégat particulaire a une taille comprise entre 30 et 500 nm.

13. Agrégat selon la revendication 12, dans lequel les antigènes comprennent le HBsAg uniquement.

14. Agrégat selon la revendication 12, dans lequel les antigènes comprennent le HBsAg en combinaison avec d'autres antigènes.

15. Agrégat selon la revendication 14, dans lequel les autres antigènes sont sélectionnés dans le groupe consistant en l'antigène de la nucléocapside du virus de l'hépatite B (HBcAg), l'antigène de la nucléocapside du virus de l'hépatite C, l'antigène de la nucléocapside du virus de l'immunodéficience humaine de type 1 ou 2, et la protéine de la membrane externe de *Neisseria meningitidis.*

16. Agrégat selon l'une quelconque des revendications 12 à 15, où l'agrégat comprend un adjuvant ayant une nature hydrophobe totale ou partielle.

17. Formulation de vaccin comprenant un agrégat hautement immunogène tel que défini dans l'une quelconque des revendications 12 à 16, avec un adjuvant, un agent stabilisant ou un agent conservateur.

18. Formulation de vaccin selon la revendication 17, destinée à une application par voie systémique ou muqueuse.
